# EUROPÄISCHE PATENTSCHRIFT

(11) **EP 1 867 797 B1**
(45) Veröffentlichungstag und Bekanntmachung des Hinweises auf die Patenterteilung: **09.03.2011**
(21) Anmeldenummer: 07090105.3
(22) Anmeldetag: 13.04.2007
(51) Int. Cl.: E04B 1/92, B32B 33/00, B32B 27/20

(54) **Kunststoffdampfbremsfolie zum Schutz vor elektromagnetischer Strahlung in Innenräumen**
Plastic vapor barrier film for protecting indoor spaces against electromagnetic radiation
Film plastique pare-vapeur pour protection d'espaces intérieurs contre les rayonnements électromagnétiques

(30) Priorität: 15.06.2006 DE 202006009566 U
(43) Veröffentlichungstag der Anmeldung: 19.12.2007
(73) Patentinhaber: Orbita-Film GmbH, 06369 Weissandt-Gölzau (DE)
(72) Erfinder: Fiedler, Steffen, 06114 Halle/Saale (DE); Schröter, Irene, 06369 Dohndorf (DE); Baumann, Hans-Joachim, 06366 Köthen (DE)
(74) Vertreter: Wablat, Wolfgang

(56) Entgegenhaltungen:
- EP-A- 1 587 353
- EP-A- 1 727 201
- DE-A1- 10 349 170
- JP-A- 2004 185 914
- US-A1- 2003 044 623

## Beschreibung

Die Erfindung bezieht sich auf eine Kunststoffdampfbremsfolie zum Schutz vor elektromagnetischer Strahlung in Innenräumen.

Zur Vermeidung von durch Wasserdamnfdiffusion und Tauwasserbildung bedingten Schäden an Dach- und Wandkonstruktionen von Gebäuden werden an der Rauminnenseite, insbesondere als innenseitiger Abschluss der Wärmedämmung, Kunststoffdampfbremsfolien aus Polyethylen, Polypropylen oder Polyamid angebracht. Bei einer beispielsweise aus Sparren, Dachlatten und Ziegeln gebildeten Dachkonstruktion schließen sich an eine an den Sparren befestigte diffusionsoffene Unterspannbahn zunächst eine Wärmedämmschicht und dann eine Kunststoffdampfbremsfolie an. Gleichermaßen können Kunststoffdampfbremsfolien in Wand- und Deckenkonstruktionen von Gebäuden eingebunden sein.

Es ist bekannt, dass die durch elektrische Geräte, Leitungen und funktechnische Sendeanlagen, insbesondere durch die Aufstellung von Mobilfunkstationen in Wohnnähe, erzeugten elektromagnetischen Strahlungen und Felder durch Strahlungsabsorption biologische Wirkungen auf den menschlichen Organismus in Form von Gewebeerwärmung und intrazellulären Prozessen oder der Beeinflussung des Hormonsystems, des Herz-Kreislauf-Systems und biologischer Ströme haben können. Die künstliche elektromagnetische Strahlenbelastung, die häufig weit über der natürlicherweise vorhandenen elektromagnetischen Strahlung liegt, verursacht bei immer mehr Menschen nervöse Beschwerden, Konzentrationsschwierigkeiten, Kopfschmerzen, unzureichende Schlafqualität, Vitalitätsverlust und eine verringerte seelische und körperliche Belastbarkeit - die typischen Symptome für Elektrostress. Ein Schutz vor außerhalb von Gebäuden erzeugten technischen Strahlen im Gebäudeinneren ist bisher durch die aufwändige Anbringung von Metallnetzen oder eines Innenanstrichs mit einer Graphitfarbe möglich.

Bekannt ist z.B. aus DE 103 49 170 A1, CH 659 755 A5 und WO 05/098 965 A2 die Verwendung von mit einer separaten elektrisch leitenden metallischen Schicht versehenen, insbesondere verklebten Kunststoffdampfbremsfolien. Abgesehen von dem hohen Fertigungsaufwand sind derart ausgebildete Kunststoffdampfbremsfolien insofern nachteilig, als die zu schützenden Gebäuderäume auch gegenüber der gewünschten, für die moderne Kommunikationstechnik erforderlichen Strahlung abgeschirmt sind und somit die vollständige Gebrauchsfähigkeit dieser Technik nicht gewährleistet ist. Andererseits wird die im Inneren eines derart abgeschirmten Raums erzeugte elektromagnetische Strahlung in erheblichem Umfang bis zu 30% reflektiert und dadurch deren Wirkung auf den menschlichen Organismus verstärkt. Besonders nachteilig kann sich in diesem Fall die Benutzung von Handys, schnurlosen Telefonen oder drahtlosen LAN-Anlagen auf den menschlichen Organismus auswirken, die in einem derart abgeschirmten Raum mit höchster, zudem am Kopf des Benutzers wirksamer Leistung betrieben werden.

Ein weiterer Nachteil der mit einer zum Strahlenschutz mit einer metallischen Beschichtung kaschierten Kunststoffdampfbremsfolie besteht in den aufwändigen Maßnahmen des wertstofflichen Recyclings, da vor der für die Entsorgung erforderlichen Granulierung der Kunststoffdampfbremsfolie zunächst die metallische Beschichtung entfernt werden muss.

Der Erfindung liegt die Aufgabe zugrunde, eine Kunststoffdampfbremsfolie, mit Strahlenschutzwirkung anzugeben, die mit geringem Aufwand hergestellt und wieder entsorgt werden kann und in Innenräumen einen ausreichenden Schutz des menschlichen Organismus gegenüber technischer Strahlung gewährleistet und zugleich eine ungestörte Nutzung von auf Strahlung basierender Kommunikationstechnik gewährleistet.

Erfindungsgemäß wird diese Aufgabe mit einer gemäß den Merkmalen des Anspruchs 1 ausgebildeten Kunststoffdampfbremsfolie dadurch gelöst, dass das elektrisch leitfähige Mittel aus verteilt in die Kunststoffdampfbremsfolie eingebetteten Partikeln besteht, die ein die Strahlung harmonisierendes Filter bilden und die derart in die Kunststoffdampfbremsfolie eingebettet sind, dass diese keine elektrisch leitfähige Schicht oder Schichten bilden. Vorteilhafte Weiterbildungen der Erfindung sind Gegenstand der Unteransprüche.

Der Grundgedanke der Erfindung besteht in der Ausbildung einer gleichzeitig als Dampfsperre fungierenden Kunststoffdampfbremsfolie mit in diese in einer bevorzugten Konzentration zwischen 0,5 und 10 % eingebetteten elektrisch leitfähigen Partikeln, die ein die Strahlung harmonisierendes Filter bilden. Das heißt, die elektromagnetischen Strahlen werden nicht vollständig, sondern nur teilweise sowie in unterschiedlichen Richtungen und auch zwischen den leitfähigen Partikeln reflektiert. Um diese Wirkung zu erzielen, ist dazu keine elektrisch leitfähige Schicht erforderlich. Der Fachmann weiß, dass man von Leitfähigkeit bei Werten des Oberflächenwiderstandes von 10⁵ Ohm spricht. Im Gegensatz dazu weisen die Kunststoffdampfbremsfolien nach der Erfindung Oberflächenwiderstände von 10¹⁰ Ohm auf. In umfangreichen Untersuchungen mit der erfindungsgemäßen, eine Elektrosmogschutzfolie bildenden Kunststoffdampfbremsfolie wurde festgestellt, dass eine sogenannte Harmonisierung der elektromagnetischen Strahlung stattfindet, so dass die harmonisierte Strahlung von den Probanden nicht mehr als störend empfunden wurde. Die Intensität der Strahlen, die für die Funktion von auf der Basis elektromagnetischer Strahlung betriebener und in erfindungsgemäß abgeschirmten Räumen aufgestellter Geräte erforderlich ist, wurde hingegen nicht beeinträchtigt. Kabellose Haustelefone oder Handys können in den mit der erfindungsgemäßen Kunststoffdampfbremsfolie abgeschirmten Innenräumen ohne zusätzliche Strahlenbelastung genutzt werden, da die von diesen Geräten ausgehenden elektromagnetischen Strahlen die Folie passieren können. Somit können die betreffenden Geräte, z.B. ein Handy, mit geringer Sendeleistung betrieben werden.

Die erfindungsgemäße Kunststoffdampfbremsfolie kann in einer Schicht oder durch Koextrusion auch mehrschichtig - mit bekannten Gieß- oder Blasverfahren auf einfache Weise hergestellt werden, da das aufwändige Auftragen einer elektrisch leitfähigen Beschichtung entfällt. Die elektrisch leitfähigen Partikel, die bereits in dem in den Extruder eingebrachten Ausgangsmaterial enthalten sind, können in einer Schicht oder auch in zwei oder mehreren Schichten gegebenenfalls auch in unterschiedlicher Konzentration und/oder mit unterschiedlichen leitfähigen Stoffen enthalten sein, wobei keine elektrische Leitfähigkeit erzielt wird und auch nicht für die Funktion der Kunststoffdampfbremsfolie benötigt wird. Ein weiterer Vorteil ergibt sich aus der einfachen Entsorgung der erfindungsgemäßen Kunststoffdampfbremsfolie, da deren Regranulierung ohne vorhergehendes Entfernen einer Reflexionsschicht durchgeführt werden kann.

Bei der Untersuchung der erfindungsgemäßen Kunststoffdampfbremsfolie wurden des Weiteren eine erhöhte Reflexion von Wärmestrahlung und damit als weiterer Vorteil eine zusätzliche wärmedämmende Wirkung festgestellt.

In Ausgestaltung der Erfindung bestehen die elektrisch leitfähigen Partikel aus Metallen, vorzugsweise Aluminium, Kupfer, Silber oder Gold, oder Metalllegierungen oder anderen leitfähigen Stoffen oder auch aus Kombinationen aus diesen. Beispielsweise kann die Kunststoffdampfbremsfolie auch einen leitfähigen Ruß enthalten. Darüber hinaus kann die Kunststoffdampfbremsfolie in der mindestens einen Schicht auch ein Flammen hemmendes Material enthalten.

Gemäß einem weiteren Merkmal der Erfindung liegt die Größe der elektrisch leitfähigen Partikel zwischen 100nm und 50µm, bevorzugt aber zwischen 10µm und 30µm. Der Dickenbereich kann zwischen 5µm und 1000 µm variieren.

Die erfindungsgemäße Kunststoffdampfbremsfolie wird als Dampfsperre und zum Strahlenschutz im Wand-, Decken- und Dachbereich von Gebäuden eingesetzt und kann gleichermaßen auch bei Wohnwagen, Bauwagen oder Zelten verwendet werden.

Die erfindungsgemäße Kunststoffdampfbremsfolie wird nachfolgend in einem Ausführungsbeispiel näher erläutert:

Eine durch Koextrusion und ein Blasverfahren in herkömmlicher Weise hergestellte dreischichtige Kunststoffdampfbremsfolie aus Polvethvlen mit geringer Dichte und einer Gesamtdicke der Folie von 100µm enthält in der ersten und dritten Schicht elektrisch leitfähige Partikel aus Aluminium und zwar jeweils in einem Anteil von 10%, während in der zweiten Schicht in einem Anteil von 10% ein flammenhemmendes Material enthalten ist. Die Dicke der ersten und dritten Schicht beträgt jeweils 20µm, während die zweite Schicht eine Dicke von 60µm aufweist. Keine Schicht der Kunststoffdampfbremsfolie wird jedoch dabei elektrisch leitfähig ausgebildet. Die so ausgebildete Kunststoffdampfbremsfolie wurde hinsichtlich ihrer harmonisierenden Wirkung auf die von einem DECT-Telefon erzeugten Strahlen untersucht. Die durch das DECT-Telefon erzeugte Strahlenbelastung entspricht etwa der einer Mobilfunk-Sendeanlage. Die Untersuchung erfolgte bei drei Probanden durch Messung der Hauttemperatur, der Herzfrequenz und des Blutvolumenstroms unter Strahlenbelastung mit und ohne die oben beschriebene Dampfsperrfolie. Ohne den Schutz durch die Elektrosmog-Schutzfolie konnten bei den drei Testpersonen eine deutliche Änderung der Signale des vegetativen Nervensystems und eine Stresserhöhung festgestellt werden, was sich in einer Erhöhung der Hautleitwerte und der Anzahl der Herzschläge/Minute sowie in einer Reduzierung des Blutvolumenstroms trotz erhöhter Herzfrequenz ausdrückt. Die Probanden haben nach ihren Aussagen die harmonisierende Wirkung der Elektrosmog-Schutzfolie auch körperlich gespürt. Die Untersuchungen durch ein auf dem Gebiet der Elektrosmog-Forschung anerkanntes Prüflabor haben gezeigt, dass die verwendete Dampfbremsfolie eine positive Veränderung des Potenzials auf der Ebene der Energiekomponente hochfrequenter elektromagnetischer Strahlung bewirkt. Eine Verringerung der ebenfalls gemessenen Strahlunasdichte der eingesetzten Strahlungsquelle wurde nicht festgestellt, so dass auch unter Schutz der Elektrosmogschutzfolie von einer uneingeschränkten Funktionsfähigkeit von auf der Basis elektromagnetischer Felder arbeitender Kommunikationsgeräte ausgegangen werden kann.

Bei der erfindungsgemäßen Kunststoffdampfbremsfolie sind keine elektrisch leitfähige Schicht und daher auch keine Erdung oder Verbindung einzelner Bahnen der Folie erforderlich, um die gewünschte Wirkung zu erreichen, d.h. die Gewährleistung eines ausreichenden Schutzes des menschlichen Organismus gegenüber technischer Strahlung und zugleich eine ungestörte Nutzung von auf Strahlung basierender Kommunikationstechnik. Es zeigt sich auch eine vorteilhafte Wirkung auf das menschliche Wohlbefinden ohne eine messbare Reduzierung der für moderne Kommunikationsmittel notwendigen elektromagnetischen Wellen.

## Patentansprüche

1. Kunststoffdampfbremsfolie für Innenräume, zum Schutz des menschlichen Organismus vor elektromagnetischer Strahlung durch ein mit der Folie verbundenes, elektrisch leitfähiges Mittel,
**dadurch gekennzeichnet,**
**dass** das elektrische leitfähige Mittel aus verteilt in die Kunststoffdampfbremsfolie eingebetteten Partikeln besteht, die ein die Strahlung harmonisierendes Filter bilden und die derart in die Kunststoffdampfbremsfolie eingebettet sind, dass diese keine elektrisch leitfähige Schicht oder Schichten bilden.

2. Kunststoffdampfbremsfolie nach Anspruch 1, **dadurch gekennzeichnet, dass** die elektrisch leitfähigen Partikel aus Metall bestehen.

3. Kunststoffdampfbremsfolie nach Anspruch 2, **dadurch gekennzeichnet, dass** die leitfähigen Partikel aus Aluminium, Kupfer, Silber, Gold oder anderen leitfähigen Metallen oder Metalllegierungen oder sonstigen leitfähigen Stoffen oder Gemischen aus diesen bestehen.

4. Kunststoffdampfbremsfolie nach einem der Ansprüche 1 bis 3, **dadurch gekennzeichnet, dass** die Größe der elektrisch leitfähigen Partikel zwischen 100nm und 50µm liegt.

5. Kunststoffdampfbremsfolie nach Anspruch 4, **dadurch gekennzeichnet, dass** die Größe der elektrisch leitfähigen Partikel zwischen 10 µm und 30 µm liegt.

6. Kunststoffdampfbremsfolie nach Anspruch 1, **dadurch gekennzeichnet, dass** als Kunststoff Polyethylen, Polypropylen, Polyamid oder ein anderes für Dampfbremsfolien übliches Material oder eine Kombination aus diesen vorgesehen ist.

7. Kunststoffdampfbremsfolie nach Anspruch 6, dass in den Kunststoff ein leitfähiger Ruß eingemischt ist.

8. Kunststoffdampfbremsfolie nach Anspruch 6, **dadurch gekennzeichnet, dass** in den Kunststoff ein Flammen hemmendes Material eingebunden ist.

9. Kunststoffdampbremsfolie nach Anspruch 1, **dadurch gekennzeichnet, dass** der Anteil der elektrisch leitfähigen Partikel in einem einschichtigen Kunststoff zwischen 0,5 und 10% liegt.

10. Kunststoffdampfbremsfolie nach Anspruch 1, **dadurch gekennzeichnet, dass** sie aus zwei oder mehreren Schichten aus gleichen oder unterschiedlichen Kunststoffen besteht, wobei die elektrisch leitfähigen Partikel in mindestens eine der Kunststoffschichten gleichmäßig verteilt eingebettet sind.

11. Kunststoffdampfbremsfolie nach Anspruch 10, **dadurch gekennzeichnet, dass** die leitfähigen Partikel in den zwei oder mehreren Schichten in gleicher oder unterschiedlicher Menge und/oder Größe angeordnet sind.

12. Kunststoffdampfbremsfolie nach Anspruch 10 oder 11, **dadurch gekennzeichnet, dass** eine dreischichtige Kunststoffdampfbremsfolie aus Polyethylen mit geringer Dichte vorgesehen ist, bei der die Dicke der ersten und dritten Schicht jeweils etwa 20µm und der zweiten, mittleren Schicht etwa 60µm beträgt, so dass die Gesamtdicke etwa 100µm beträgt, und die in der ersten und dritten Schicht elektrisch leitfähige Partikel aus Aluminium jeweils in einem Anteil von etwa 10% und in der zweiten, mittleren Schicht ein flammenhemmendes Material mit einem Anteil von etwa 10% enthält.

13. Kunststoffdampfbremsfolie nach einem der Ansprüche 1 bis 12, **dadurch gekennzeichnet, dass** diese im Dachbereich sowie im Wand- und Deckenbereich unter Putz oder auf Putz unter einer Zierputzschicht bzw. Tapete angeordnet ist.

## Claims

1. A plastic vapour barrier sheet for interior spaces to protect the human organism from electromagnetic radiation through an electrically conductive means connected to the sheet,
**characterised in that**
the electrically conductive means comprises particles embedded throughout the plastic vapour barrier sheet, which create a radiation-harmonising filter and are embedded in the plastic vapour barrier sheet in such a way that they do not form an electrically conductive layer or layers.

2. The plastic vapour barrier sheet according to claim 1, **characterised in that** the electrically conductive particles are made of metal.

3. The plastic vapour barrier sheet according to claim 2, **characterised in that** the conductive particles are made of aluminium, copper, silver, gold or other conductive metals or metal alloys or other conductive materials or mixtures of these.

4. The plastic vapour barrier sheet according to one of the claims 1 to 3, **characterised in that** the size of the electrically conductive particles is between 100 nm and 50 µm.

5. The plastic vapour barrier sheet according to claim 4, **characterised in that** the size of the electrically conductive particles is between 10 µm and 30 µm.

6. The plastic vapour barrier sheet according to claim 1, **characterised in that** polyethylene, polypropylene, polyamide or another customary vapour barrier sheet material or a combination of these is envisaged as the plastic.

7. The plastic vapour barrier sheet according to claim 6, (**characterised in**) that a conductive soot is mixed into the plastic.

8. The plastic vapour barrier sheet according to claim 6, **characterised in that** a flame-retardant material is incorporated in the plastic.

9. The plastic vapour barrier sheet according to claim 1, **characterised in that** the proportion of electrically conductive particles in a single-layer plastic is between 0.5 and 10%.

10. The plastic vapour barrier sheet according to claim 1, **characterised in that** it consists of two or more layers of identical or different plastics, wherein the electrically conductive particles are evenly embedded throughout at least one of the plastic layers.

11. The plastic vapour barrier sheet according to claim 10, **characterised in that** the conductive particles are disposed in the two or more layers in an identical or different quantity and/or size.

12. The plastic vapour barrier sheet according to claim 10 or 11, **characterised in that** a triple-layer plastic vapour barrier film made from low-density polyethylene is envisaged, in which the thickness of the first and third layer is roughly 20 µm in each case and the thickness of the second, middle layer is roughly 60 µm, so that the total thickness is roughly 100 µm, and which contains electrically conductive particles made of aluminium in a proportion of roughly 10 % in each case in the first and third layer and a flame-retardant material with a proportion of roughly 10 % in the second, middle layer.

13. The plastic vapour barrier sheet according to one of the claims 1 to 12, **characterised in that** this is disposed in the roof space and in the wall and ceiling space under the plaster or on the plaster beneath a decorative layer of plaster or wallpaper.

## Revendications

1. Film pare-vapeur en matière plastique pour la protection de l'organisme humain contre les rayonnements électromagnétiques par un moyen conducteur électrique, relié au film, **caractérisé en ce que** le moyen conducteur électrique consiste dans des particules noyées dans le film pare-vapeur en matière plastique, qui forment un filtre harmonisant le rayonnement et qui sont noyées dans le film pare-vapeur en matière plastique de sorte que celui-ci ne forme aucune couche(s) conductrice(s).

2. Film pare-vapeur en matière plastique selon la revendication 1, **caractérisé en ce que** les particules conductrices d'électricité sont en métal.

3. Film pare-vapeur en matière plastique selon la revendication 2, **caractérisé en ce que** les particules conductrices d'électricité sont en aluminium, en cuivre, en argent, en or ou en d'autres métaux ou alliages métalliques conducteurs ou en d'autres matières conductrices ou mélanges de ces derniers.

4. Film pare-vapeur en matière plastique selon l'une quelconque des revendications 1 à 3, **caractérisé en ce que** la dimension des particules conductrices d'électricité se situe entre 100 nm et 50 µm.

5. Film pare-vapeur en matière plastique selon la revendication 4, **caractérisé en ce que** la dimension des particules conductrices d'électricité se situe entre 10 µm et 30 µm.

6. Film pare-vapeur en matière plastique selon la revendication 1, **caractérisé en ce qu'**en tant que matière plastique, il est prévu du polyéthylène, du polypropylène, du polyamide ou une autre matière usuelle pour les films pare-vapeur ou une association de ces derniers.

7. Film pare-vapeur en matière plastique selon la revendication 6, **caractérisé en ce qu'**un noir de carbone conducteur est mélangé à la matière plastique.

8. Film pare-vapeur en matière plastique selon la revendication 6, **caractérisé en ce qu'**une matière ignifuge est incorporée dans la matière plastique.

9. Film pare-vapeur en matière plastique selon la revendication 1, **caractérisé en ce que** la part des particules conductrices d'électricité dans une matière plastique monocouche se situe entre 0,5 et 10 %.

10. Film pare-vapeur en matière plastique selon la revendication 1, **caractérisé en ce qu'**il consiste dans deux ou dans plusieurs couches dans des matières plastiques identiques ou différentes, les particules conductrices d'électricité étant noyées en étant régulièrement réparties dans au moins l'une des couches en matière plastique.

11. Film pare-vapeur en matière plastique selon la revendication 10, **caractérisé en ce que** les particules conductrices d'électricité dans les deux ou plusieurs couches sont disposées en quantité et/ou dimension égales ou différentes.

12. Film pare-vapeur en matière plastique selon la revendication 10 ou 11, **caractérisé en ce qu'**il est prévu un film pare-vapeur en matière plastique à trois couches, en polyéthylène de faible densité, sur lequel l'épaisseur de la première et de la troisième couche est d'environ 20 µm pour chacune et celle de la deuxième couche centrale est d'environ 60 µm, de sorte que l'épaisseur totale soit de 100 µm et qui contient les particules conductrices dans la première et dans la troisième couche dans une part de 10% environ pour chacune et qui contient dans la deuxième couche centrale une matière ignifuge dans une part de 10 % environ.

13. Film pare-vapeur en matière plastique selon l'une quelconque des revendications 1 à 12, **caractérisé en ce qu'**il est disposé dans la zone du toit, ainsi que dans la zone des murs et des plafonds, soit sous enduit ou sur enduit, sous une couche de crépi décoratif ou un papier peint.
